Europäisches Patentamt

European Patent Office

Office européen des brevets

(19)

(11) **EP 0 727 434 B1**

(12) **EUROPÄISCHE PATENTSCHRIFT**

(45) Veröffentlichungstag und Bekanntmachung des Hinweises auf die Patenterteilung:
**25.08.1999  Patentblatt 1999/34**

(51) Int Cl.⁶: **C07K 14/745**

(21) Anmeldenummer: **96890019.1**

(22) Anmeldetag: **12.02.1996**

(54) **Reinigung von Gewebefaktor**

Purification of tissue factor

Purification de facteur tissulaire

(84) Benannte Vertragsstaaten:
**AT BE CH DE DK ES FR GB IT LI NL SE**

(30) Priorität: **15.02.1995  AT 27595**

(43) Veröffentlichungstag der Anmeldung:
**21.08.1996  Patentblatt 1996/34**

(73) Patentinhaber: **IMMUNO Aktiengesellschaft
A-1221 Wien (AT)**

(72) Erfinder:
• **Lang, Hartmut, Dr.
A-2560 Grillenberg (AT)**
• **Moritz, Berta, Dr.
A-1030 Wien (AT)**

(74) Vertreter: **Pawloy, Peter Michael, Dipl.-Ing. et al
Patentanwälte
Sonn, Pawloy, Weinzinger & Wolfram
Riemergasse 14
1010 Wien (AT)**

(56) Entgegenhaltungen:
EP-A- 0 603 671          EP-A- 0 670 331
WO-A-92/08479          WO-A-93/07492

• JOURNAL OF BIOLOGICAL CHEMISTRY (MICROFILMS), Bd. 256, Nr. 16, 25.August 1981, MD US, Seiten 8324-8331, XP002005090 R BACH ET AL.: "Purification and characterization of bovine tissue factor"

**Beschreibung**

Die Erfindung betrifft eine aktive Gewebefaktor-Präparation, ein Verfahren zu deren Herstellung sowie ein diese Präparation enthaltendes Reagens.

Der Gewebefaktor (tissue factor) ist die Proteinkomponente (Apoprotein) eines integralen Membranlipoproteins, des Gewebethromboplastins, auch als Faktor III bezeichnet, und spielt im extrinsischen Teil der Blutgerinnung eine bedeutende Kofaktor-Rolle.

Das Gewebethromboplastin stellt einen Rezeptor für den Faktor VII des Blutgerinnungssystems dar und enthält neben dem Apoprotein auch Lipide. Das Apoprotein, der Gewebefaktor, ist ein glykosyliertes Polypeptid von 263 Aminosäuren, das nahe dem carboxyterminalen Ende eine hydrophobe Sequenz von 23 Aminosäuren besitzt, mit der es in der Membran verankert ist. Seine physiologische Funktion besteht darin, als Zelloberflächenrezeptor bei Kontakt mit Blut bzw. Plasma den plasmatischen Gerinnungsfaktor VII zu binden und zu aktivieren. Für diese Gerinnungsaktivität sind aber beide Komponenten des Gewebethromboplastins notwendig, sowohl das Protein als auch die Phospholipidkomponente.

Der Komplex des Gewebefaktors mit Faktor VIIa besitzt Serinprotease-Aktivität und ist seinerseits in der Lage, die Faktoren IX und X zu aktivieren und damit die Gerinnung auszulösen.

Gewebethromboplastin wird in der Bestimmung der Thromboplastinzeit, die auch als Prothrombinzeit-Bestimmung oder Quick-Wert-Bestimmung bezeichnet wird, verwendet. Hierbei wird Zitratplasma mit einem Überschuß an Gewebethromboplastin und Calciumionen inkubiert. Gemessen wird die Zeit bis zum Eintreten der Gerinnung, die von den Gerinnungsfaktoren VII, X, V, II und Fibrinogen im Plasma abhängig ist. Die Ergebnisermittlung kann auf verschiedene Weise erfolgen. Bei einer älteren Methode dient beispielsweise eine mit verdünntem Plasma von Gesunden gewonnene Bezugskurve zur Umrechnung der gemessenen Zeit in Prozent der normalen Aktivität. Für Plasmen oral antikoagulierter Patienten wurde zur Standardisierung der Prothrombinzeit gemäß den WHO-Richtlinien von 1983 die sogenannte INR (INR = International Normalized Ratio) definiert, welche sich aus folgender Formel ergibt:

$$INR = \left(\frac{SekundenPatientenplasma}{SekundenNormalplasma}\right)^{ISI}$$

ISI ist der "International Sensitivity Index", der die Sensitivität der Thromboplastine gegenüber dem 1. WHO-Referenzthromboplastin beschreibt und einen Wert zwischen 0,8 und 2,5 aufweist.

Das Gewebethromboplastin kann aus unterschiedlichen Gewebearten, wie beispielsweise Hirn, Lunge oder Plazenta tierischen oder humanen Ursprungs, isoliert werden. Dabei können prinzipiell zwei Methoden zur Anwendung kommen.

Bei der einen Methode erhält man durch Aufschluß bzw. Extraktion des entsprechenden Gewebes ein grob gereinigtes Gewebethromboplastin, wobei das Membranprotein als Ganzes, also inklusive der Lipidmoleküle, mit denen es in vivo assoziiert ist, isoliert wird.

Das so gewonnene Thromboplastin weist aber je nach Art des verwendeten Ausgangsmaterials und auch saisonal bedingt wechselnde Mengen an Gewebefaktor- und Phospholipidanteilen auf. Hierdurch ist eine hohe Variabilität von Lot zu Lot gegeben. Die Rohextrakte können zusätzlich durch andere Gerinnungsfaktoren verunreinigt sein. All dies führt gerade bei Verwendung des Thromboplastins zur Herstellung von Diagnostika zu einer schwierigen Standardisierbarkeit und beeinflußt die erhaltene Thromboplastinzeit.

Bei der zweiten prinzipiellen Methode zur Reinigung von Gewebethromboplastin wird das isolierte Apoprotein, der Gewebefaktor als solches, erhalten. Da das isolierte Protein fast keine Aktivität mehr aufweist, ist eine Reaktivierung notwendig, um das Protein erneut in eine Lipid-Membran zu integrieren. Die Reaktivierung erfolgt im allgemeinen durch Zugabe von Phospholipiden, und dieser Prozeß wird als Relipidisierung bezeichnet.

Ein Beispiel für diese zweite Methode wird etwa in E. Bjorklid et al., Biochem.J. 165 (1977), S. 89-96, "Purification and Some Properties of the Protein Component of Tissue Thromboplastin from Human Brain", beschrieben, wobei die Reinigung des Gewebefaktors aus humanem Hirn durch Extraktion mit Natriumdesoxycholat und anschließender Gelfiltration vorgenommen wird. Dadurch wird der Gewebefaktor zwar frei von Phospholipiden erhalten, jedoch ist der Gewebefaktor nur grob gereinigt. Für die Herstellung von Thromboplastin erfolgt die Relipidisierung durch Zusatz von Phospholipiden, wie Phosphatidylcholin, Phosphatidylethanolamin, Phosphatidylserin und Mischungen derselben in Anwesenheit von CaCl$_2$.

Auch in Bach R. et al., The Journal of Biological Chemistry, Bd. 256 (16) (1981), S. 8324-8331, "Purification and Characterization of Bovine Tissue Factor", wird eine Reinigung des Gewebefaktors unter Verwendung von Immunadsorption und anschließender Relipidisierung mit einer Mischung unterschiedlicher, aus bovinem Gehirn gewonnener Phospholipide in Gegenwart von Schwermetallionen wie beispielsweise Cd$^{2+}$ beschrieben. Es wurde gefunden, daß es für eine effiziente Relipidisierung essentiell ist, diese Schwermetallionen zu verwenden, wogegen eine Relipidisierung, welche ohne Cd$^{2+}$, sondern lediglich in Gegenwart von Ca$^{2+}$ durchgeführt worden ist, nicht zufriedenstellend sein soll.

Carson St. D. et al., Thrombosis and Haemostasis, Bd. 44 (1980), S. 12-15, "Lipid Activation of Coagulation Factor III Apoprotein (Tissue Factor) - Reconstitution of the Protein Membrane Complex", beschreiben die Rekonstitution von Thromboplastin aus Gewebefaktor, der aus humaner Plazenta mittels Extraktion und anschließender Reinigung an Phenyl-Sepharose und Con A-Sepharose erhalten wurde, mit Phospholipiden in Gegenwart von Cadmiumionen.

Neben diesen Methoden, Gewebefaktor aus konventionellen Quellen zu erhalten, sind auch Verfahren zur rekombinanten Herstellung bekannt und beispielsweise in der EP-A-O 278 776 oder in der WO 93 07492 beschrieben. Jedoch können selbst geringfügige Unterschiede der rekombinanten Präparation vom biogenen Material, wie z.B. Glykosylierung, Verunreinigungen mit Substanzen aus dem Expressionssystem (Fremdproteine, Nukleinsäuren, etc.), ungenaue proteolytische Spaltung,..., zu verfälschten Ergebnissen führen.

Die im Stand der Technik beschriebenen Verfahren machen üblicherweise für eine optimale Relipidisierung, die eine der wesentlichen Voraussetzungen für ein zufriedenstellendes Produkt ist, Zusätze von Schwermetallkationen, wie beispielsweise Cadmium, notwendig. Aufgrund der Toxizität von Schwermetallionen im allgemeinen ist der Zusatz solcher Stoffe in biologisch angewandten Präparationen jedoch nicht wünschenswert.

Ein weiterer Nachteil bei den bekannten Verfahren ergibt sich durch die Zugabe von Phospholipiden undefinierter oder nicht optimaler Zusammensetzungen, da sowohl Art als auch Reinheitsgrad der Lipide von entscheidender Bedeutung sind und stark variieren können.

Ein weiteres Problem bildet die im allgemeinen starke Trübung der Thromboplastin enthaltenden Lösungen, welche daher für den diagnostischen Einsatz in einem Gerinnungsreagens problematisch sind, da solche Diagnosen meist mit Hilfe von optischen Bestimmungsmethoden, beispielsweise über Trübheitsmessung, erfolgen.

Der vorliegenden Erfindung liegt daher die Aufgabe zugrunde, einen gut standardisierbaren aktiven Gewebefaktor aus konventionellen Quellen sowie ein Verfahren zu dessen Herstellung zur Verfügung zu stellen, um diese Nachteile zu vermeiden, welche bei den im Stand der Technik bekannten Produkten und Methoden gegeben sind. Ein weiteres Ziel liegt in der Schaffung eines Thromboplastinreagens und eines Reagens zur Bestimmung von Faktoren der extrinsischen Gerinnung.

Die erfindungsgemäße Aufgabe wird durch ein Verfahren zur Herstellung einer aktiven Gewebefaktor-Präparation gelöst, das durch die folgenden Schritte gekennzeichnet ist:

- Extrahieren eines biogenen Ausgangsmaterials, welches den Gewebefaktor enthält, in einem Tensid-haltigen Medium und Gewinnen eines Gewebefaktor-haltigen Extrakts,
- Reinigen des Gewebefaktor-haltigen Extrakts durch Affinitätschromatographie unter Verwendung von immobilisierten Antikörpern, welche gegen den Gewebefaktor gerichtet sind,
- Abtrennen von vorhandenem Tensid und
- Relipidieren des Gewebefaktors durch Zugabe von hochgereinigten Phospholipiden in einem Medium, welches keine Schwermetallionen aufweist.

Die Extraktion des biogenen Ausgangsmaterials erfolgt mit hierfür gängigen Substanzen, wie beispielsweise Natriumdesoxycholat, Aceton, Kohlenwasserstoffen, niederen Alkoholen oder Mischungen davon. Als Tensid-haltige Medien für die Extraktion des biogenen Gewebes werden nichtionische oder zwitterionische Detergentien, wie beispielsweise solche aus der Triton®-X-Reihe (Polyoxyethylen-p-t-octylphenole, Polyethylenglycol-p-isooctylphenylether), Tween®-Detergentien (Polysorbate, Polyoxyethylene) oder andere (siehe z.B. Römpp, Chemielexikon, 9. Auflage, Seiten 4495 ff.) verwendet.

Für die affinitätschromatographische Reinigung können polyklonale oder monoklonale Antikörper mit einer Affinität für Gewebefaktor verwendet werden; vorzugsweise werden Antikörper der gleichen Spezies wie für das Ausgangsmaterial für die Extraktion des Gewebefaktors verwendet, besonders bevorzugt werden monoklonale Antikörper verwendet. Poly- und monoklonale Antikörper mit einer Affinität für Gewebefaktor werden nach üblichen, in der Literatur beschriebenen Methoden erhalten (siehe beispielsweise Morrissey J.H. et al., Thromb.Res. 50, 481, 1988).

Die Elution des Gewebefaktors von der affinitätschromatographischen Säule kann mit Puffern hoher Salzkonzentration vorgenommen werden, die gegebenenfalls auch chaotrope Stoffe, wie beispielsweise Thiocyanat enthalten.

Das nach der Elution gegebenenfalls vorhandene Tensid kann durch eine Fällung des Gewebefaktors oder durch spezielle Gele, z.B. für die "Umkehrphasen-Chromatographie", mit einer hohen Affinität für Tenside, abgetrennt werden, um reinen Gewebefaktor zu erhalten.

Der gereinigte Gewebefaktor wird mit hochgereinigten Phospholipiden ohne Zusatz von Schwermetallionen assoziiert, wobei dieser Prozeß sowohl mit als auch ohne $Ca^{2+}$-Ionen durchgeführt werden kann. Unter hochgereinigten Phospholipiden werden solche Stoffe verstanden, die aus tierischen oder pflanzlichen Quellen stammen und mittels chromatographischer Verfahren oder chemischer Methoden gereinigt werden. Die Phospholipide werden bevorzugt in vesikulärer Form zugegeben.

Als Phospholipide kommen Stoffe, vorzugsweise chemischer Reinheit, wie beispielsweise Phosphatidylcholin, Phosphatidylserin, Phosphatidylethanolamin, Phosphatidylglycerin, Spingomyelin, Phosphatidylinositol, Phosphatidylsäure etc. zum Einsatz. Die Phospholipide können auch als Mischungen vorliegen, im besonderen werden die Phospholipidzusammensetzungen, aber auch die Konzentration des Gewebefaktors, so optimiert, daß die Thromboplastinzeit des Reagens mit Normalplasma in einem Bereich zwischen 10 s und 15 s liegt.

Eine besonders bevorzugte Mischung von Phospholipiden zur Relipidisierung des Gewebefaktors enthält 20-50 % Phosphatidylcholin, 20-50 % Phosphatidylserin, 0-20 % Phosphatidylethanolamin und 0-20 % Phosphatidylsäure. Gegebenenfalls können auch Lipide, wie beispielsweise Cholesterin, Aminosäuren, z.B. Glutaminsäure, Zucker oder einwertige Ionen, wie beispielsweise Cäsium-Ionen, zugesetzt werden.

Das Verfahren umfaßt bevorzugterweise auch einen Schritt zur Virusinaktivierung. Diese Virusinaktivierung kann nach heute gängigen Methoden erfolgen, insbesondere mit einer Hitzebehandlung,

UV-Bestrahlung, Behandlung mit Tensiden und/oder einer Behandlung mit einer chemischen Substanz, z.B. Thiocyanat. Die einzelnen Verfahrensschritte können dabei gleichzeitig (z.B. Hitzebehandlung unter gleichzeitiger Tensid-Behandlung) oder in beliebiger Reihenfolge hintereinander erfolgen. Bevorzugt erfolgt die Assoziation des Gewebefaktors durch Phospholipide in vesikulärer Form.

Gemäß einem weiteren Aspekt betrifft die vorliegende Erfindung eine aktive Gewebefaktor-Präparation von biogener Herkunft, die entweder als klare Lösung vorliegt oder in lyophilisierter Form und zu einer klaren Lösung rekonstituiert werden kann, welche Gewebefaktoren-Präparation durch das erfindungsgemäße Verfahren erhältlich ist. Die erfindungsgemäße Präparation zeichnet sich daher durch die folgenden Merkmale aus:

- sie enthält einen Gewebefaktor, welcher aus biogenem Ausgangsmaterial stammt und durch Affinitätschromatographie gereinigt ist,
- sie enthält hochgereinigte Phospholipide, und
- sie enthält keine Schwermetallionen.

Unter einer klaren Lösung wird eine Lösung verstanden, welche eine Extinktion im Vergleich zu Wasser von 0,6 oder weniger bei 700 μm und einer Schichtdicke von 1 cm aufweist.

Die Untergrenze für die Extinktion ist für den Einsatz der Präparation als Diagnosemittel essentiell. Nur eine Präparation, deren Extinktion unterhalb dieser Grenze liegt, kann als optisch klare Lösung angesehen werden. Das Vorliegen der erfindungsgemäßen Gewebefaktor-Präparation als optisch klare Lösung bietet gerade bei deren Verwendung in modernen Analysengeräten, in denen die Detektion mit optischen Einrichtungen erfolgt, einen großen Vorteil. Ein weiterer Vorteil der erfindungsgemäßen optisch klaren Präparation liegt darin, daß auch nach längerer Zeit keine Sedimentation des Gewebefaktors erfolgt.

Die erfindungsgemäße Gewebefaktor-Präparation beruht ausschließlich auf biogenem Material, ohne daß rekombinantes Material verwendet wird. Nur mit einem Gewebefaktor aus biogenem Material ist gewährleistet, daß physiologisch richtige Ergebnisse bei der Diagnose erhalten werden, da damit Probleme, welche sich durch nichtphysiologische Glykosylierung, Faltung der Proteinkette, proteolytische Prozessierung, etc. ergeben können, von vornherein nicht gegeben sind. Insbesondere kann durch den biogenen Ursprung des Gewebefaktors die Verunreinigung der Gewebefaktor-Präparation mit artfremden Proteinen oder Nukleinsäuren und mit Substanzen aus Kulturflüssigkeiten, z.B. artfremder Gewebefaktor aus Serum, das der Kulturflüssigkeit zugesetzt worden ist, vermieden werden. Als biogene Ausgangsmaterialien für den Gewebefaktor werden Gewebe wie beispielsweise Hirn, Lunge oder Plazenta humanen Ursprungs oder von Säugetieren stammend verwendet.

Der Gewebefaktor muß in einer hinreichend reinen Form in der Präparation enthalten sein, um den hohen Anforderungen an ein standardisierbares Diagnosemittel zu genügen. Es hat sich gezeigt, daß ein Gewebefaktor, welcher mittels einer affinitätschromatographischen Methode gereinigt worden ist, einen ausreichenden Reinheitsgrad aufweist, da durch eine derartige Methode gezielt solche Proteine vom Gewebefaktor abgetrennt werden können, welche normalerweise mit dem Gewebefaktor eng verbunden sind und deren Anwesenheit in einem Diagnosetest die Ergebnisse verfälschen. Die erfindungsgemäße Gewebefaktor-Präparation ist im wesentlichen frei von physiologischen Gewebefaktor-Begleitproteinen. So sind beispielsweise keine gerinnungsaktiven Faktoren II, VII, IX und X nachweisbar.

Die erfindungsgemäße Gewebefaktor-Präparation ist frei von Schwermetallionen bzw. liegt deren Konzentration unter der Nachweisgrenze von gängigen Methoden zur Bestimmung von Schwermetallionen, beispielsweise polarografischen Methoden oder der Atomabsorption; sie weist allerhöchstens den Gehalt auf, der von vornherein im Ausgangsmaterial vorhanden war. Jedenfalls liegt der Gehalt an Schwermetallionen in der erfindungsgemäßen Präparation deutlich unter den bekannten Präparationen, die durch eine Relipidisierung unter Verwendung von beispielsweise $Cd^{2+}$ hergestellt worden sind. Dadurch kann das Arbeiten mit toxischen Schwermetallionen, insbesondere mit $Cd^{2+}$-Ionen vermieden werden.

Weiters werden damit auch die möglichen Störungen, welche bei Anwesenheit einer unphysiologischen $Cd^{2+}$-Konzentration bei einer diagnostischen Methode auftreten können (z.B. eine Verzögerung der Gerinnungszeit), vermieden. Zur Herstellung eines beliebigen Reagens kann aber eine definierte Menge an Calciumionen zugesetzt werden kann, was zu einem besser standardisierbaren diagnostischen Mittel führt.

Aufgrund der beschriebenen charakteristischen Merkmale der erfindungsgemäßen Gewebefaktor-Präparation ist diese auch gemäß den internationalen Standards für Thromboplastine zur diagnostischen Verwendung standardisierbar (siehe beispielsweise WHO Expert Committee on Biological Standardization 33rd report, WHO Geneva, 1983, "Requirements for thromboplastins and plasma used to control oral anticoagulant therapy").

Als besonders geeignet hat sich ein Gewebefaktor aus konventionellen Quellen, wie z.B. aus Kaninchen oder Rind, erwiesen.

Die erfindungsgemäße Präparation weist gemäß einer bevorzugten Ausführungsform einen Tensidgehalt von weniger als 0,5%, besonders bevorzugt weniger als 0,1 Gew.-%, auf. Da sich die meisten Herstellungsmethoden für Gewebefaktor eines Verfahrensschrittes mit Tensiden bedienen, diese Tenside aber bei der Verwendung der Gewebefaktor-Präparation als Diagnosemittel stören könnten, ist eine möglichst vollständige Abtrennung der Tenside aus der Präparation vorteilhaft.

Da die erfindungsgemäße Präparation insbesondere in der Diagnose verwendet werden soll, enthält sie vorteilhafterweise einen definierten Gehalt an Calciumionen im Bereich zwischen 5 mM und 50 mM, besonders bevorzugt 10 mM bis 15 mM, sowie einen geeigneten Puffer, vorzugsweise einen Tris-NaCl-, Imidazol- oder HepesGlycin-Puffer.

Die Erfindung umfaßt auch die Verwendung der erfindungsgemäßen Gewebefaktor-Präparation zur Herstellung eines Thromboplastinreagens sowie zur Herstellung eines Reagens zur Bestimmung von Faktoren der extrinsischen Gerinnung.

Das erfindungsgemäße Thromboplastinreagens enthält neben der aktiven Gewebefaktor-Präparation auch Calciumionen in einem geeigneten Puffer. Als Calciumquelle kann beispielsweise $CaCl_2$ dienen, die Konzentration an Calciumionen sollte üblicherweise zwischen 5mM und 50mM liegen.

Die Erfindung umfaßt auch ein Set zur Herstellung einer Gewebefaktor-Präparation enthaltend die Komponenten

 a) affinitätschromatographisch gereinigten Gewebefaktor und
 b) hochgereinigte Phospholipide.

Das Set enthält vorzugsweise auch beide Komponenten in lyophilisierter Form und weiters eine wässerige Lösung frei von Schwermetallionen zur Rekonstitution.

Die Erfindung wird anhand der nachstehenden Beispiele und der dazugehörigen Zeichnungsfigur, auf die sie jedoch nicht beschränkt sein soll, noch weiter erläutert.

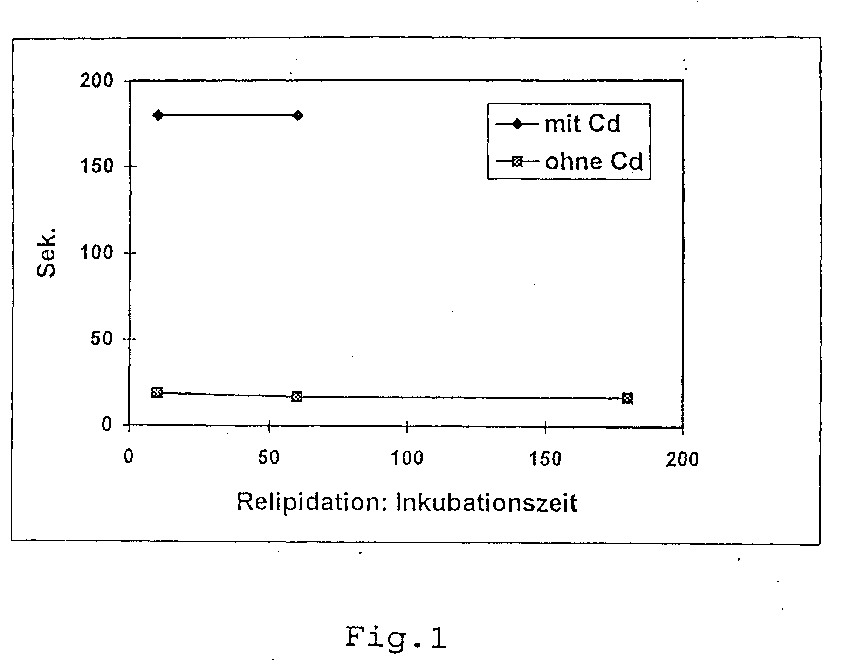

Es zeigt Fig. 1 die Relipidisierung mit und ohne $Cd^{2+}$.

### Beispiel 1: Herstellung des aktiven Gewebefaktors:

Kommerziell erhältliches Thromboplastin humanen Ursprungs (Thromborel S, Fa. Behring) wurde in destilliertem Wasser unter Zusatz von 0,1% Triton gelöst. Die Probe wurde mit immobilisiertem Anti-hTF (Anti-hTF an BrCN-Sepharose) im Batchverfahren eine Stunde am Schüttelgerät inkubiert, das Gel anschließend gut gewaschen (Waschpuffer: 6 g/l Tri-Natriumcitrat, 7 g/l NaCl, 0,1% Triton, pH: 7,35) und mit 8 ml Elutionspuffer eine 1/2 Stunde inkubiert. (Elutionspuffer: 3M Natriumthiocyanat, ev. 0,1% Triton, pH: 7,0).

Das Eluat wurde über Nacht bei +4°C dialysiert (Dialysepuffer: 10,2 g/l NaCl, 6,06 g/l Tris, ev. 1,9 g/l $CaCl_2$. $2H_2O$, pH: 7,4).

Die Relipidisierung erfolgt mit einem Teil gereinigtem Gewebefaktor, wobei das Eluat nach der Dialyse verwendet wurde, und einem Teil verdünnter Phospholipidlösung für 3 Stunden bei 37°C.

Die Phospholipidlösung weist folgende Zusammensetzung auf: 31% Phosphatidylserin, 42% Phosphatidylcholin, 7% Phosphatidylethanolamin, 10% Phosphatidylsäure, 10% Cholesterin und 60 mmol Glutaminsäure, wobei 2 mg/ml 1:20 in Dialysepuffer verdünnt werden.

Der so hergestellte aktive Gewebefaktor kann auch lyophilisiert werden. Hierfür empfiehlt sich z.B. Prionexoder Gelatinezugabe (2%) (beide Produkte von Fa. Pentapharm).

### Beispiel 2: Herstellung des Reagens:

Der in Beispiel 1 hergestellte aktive Gewebefaktor wird mit einer 10-15 mM $CaCl_2$-Lösung bei pH 6,8-7,8 versetzt und dadurch das entsprechende Reagens erhalten.

**Beispiel 3: Verwendung des Reagens zur Bestimmung der Thromboplastinzeit:**

Zur Bestimmung der Thromboplastinzeit wird die Plasmaprobe mit dem Thromboplastinreagens von Beispiel 2 in einer Küvette für 1 Minute bei 37°C inkubiert, und der Gerinnungseintritt wird mit einem Schnitger Gross-Koagulometer nach der Häkel-Methode bestimmt.

**Beispiel 4: Relipidisierung mit und ohne Cd++**

Im folgenden wurde gereinigter Gewebefaktor gegen einen Puffer ohne Ca++ dialysiert, anschließend erfolgte die Relipidisierung und die Verkürzung der Gerinnungszeit am Schnitger Gross-Koagulometer in einem Zeitintervall zwischen 0 und 180 Minuten mit und ohne Zusatz von Cadmiumionen gemessen.

a) Puffer ohne Cd++ und

c) Puffer mit Cd++

Die Effizienz der Relipidisierung mit und ohne Schwermetallionen wird durch Messung des Gerinnungseintritts bestimmt. Hiefür wurden 100 μl Plasmaprobe, 200 μl aktiver Gewebefaktor hergestellt nach Beispiel 1 und 20 μl einer $CaCl_2$-Lösung (Endkonzentration im Ansatz 10-15 mM) für 1 Minute bei 37°C inkubiert, und die Zeit bis zum Eintritt der Gerinnung am Schnitger Gross-Koagulometer bestimmt. Die Ergebnisse sind in Fig. 1 dargestellt. Der Zusatz von Cadmiumionen führt zu einer starken Verzögerung der Gerinnungszeit.

**Beispiel 5: Bestimmung der optischen Klarheit**

Verschiedene Thromboplastine (sowohl nach konventionellen Methoden gereinigte (Thromboplastin IS® von Baxter, Thromborel S® von Behring, Thrombolastin® von Diagen) als auch rekombinante Präparationen (Innovin® von Baxter, Recombiplastin® von Ortho)) wurden nach Vorschrift gelöst, in einer Quarzküvette stehengelassen, und das Spektrum bei 280 und 700 nm wurde täglich gemessen. Bei einigen Proben wurde das Auftreten eines Bodensatzes beobachtet.

Eine Übersicht zu den photometrischen Bestimmungen gibt die folgende Tabelle:

| Thromboplastin | Hersteller | Ext./Bodsatz | Tag 1 | Tag 2 | Tag 3 | Tag 6 | Tag 7 | Tag 8 |
|---|---|---|---|---|---|---|---|---|
| Thromboplastin IS | Baxter | 280 nm | 4,44 | 1,81 | 4,19 | 4,35 | 3,35 | 3,76 |
| | | 700 nm | 0,92 | 1,15 | 0,56 | 1,08 | 1,02 | 0,61 |
| | | Bodsatz | - | - | - | + | + | + |
| Thromborel S | Behring | 280nm | 4,56 | 4,73 | 4,46 | 4,52 | 4,56 | 4,52 |
| | | 700 nm | 1,28 | ,92 | 0,71 | 0,81 | 0,74 | 0,65 |
| | | Bodsatz | - | - | - | + | + | + |
| Thromboplastin | Diagen | 280 nm | 2,79 | 4,57 | 2,13 | 2,21 | 2,22 | 2,37 |
| | | 700 nm | 2,10 | 1,53 | 1,41 | 1,49 | 1,50 | 1,54 |
| | | Bodsatz | - | + | + | ++ | ++ | +++ |
| Innovin | Baxter | 280 nm | 0,247 | 0,244 | 0,242 | 0,248 | 0,258 | 2,164 |
| | | 700 nm | 0,052 | 0,052 | 0,051 | 0,052 | 0,051 | 0,051 |
| | | Bodsatz | - | - | - | - | - | - |
| Recombiplastin | Ortho | 280 nm | 0,52 | 2,84 | 0,49 | 3,01 | 2,88 | 0,49 |
| | | 700 nm | 0,067 | 0,045 | 0,037 | 0,098 | 0,037 | 0,036 |
| | | Bodsatz | - | - | - | ++ | + | + |

(fortgesetzt)

| Thromboplastin | Hersteller | Ext./ Bodsatz | Tag 1 | Tag 2 | Tag 3 | Tag 6 | Tag 7 | Tag 8 |
|---|---|---|---|---|---|---|---|---|
| erfindungsgemäßes Reagens | | 280 nm | 1,40 | 1,54 | 1,61 | 1,63 | 1,71 | 1,75 |
| | | 700 nm | 0,063 | 0,058 | 0,058 | 0,057 | 0,074 | 0,073 |
| | | Bodsatz | - | - | - | - | - | - |

Es zeigte sich deutlich, daß die nach konventionellen Methoden gereinigten Gewebefaktoren Thromboplastin IS (Baxter), Thromborel S (Behring) und Thromboplastin (Diagen) im Vergleich zu dem erfindungsgemäßen Reagens aufgrund der stärkeren Trübung durchwegs höhere Extinktionswerte bei 280 nm aufweisen. Beim Großteil der nach konventionellen Methoden gereinigten Gewebefaktoren entstand nach mehreren Tagen ein Bodensatz.

**Patentansprüche**

1. Verfahren zur Herstellung einer aktiven Gewebefaktor-Präparation, gekennzeichnet durch die folgenden Schritte:

   - Extrahieren eines biogenen Ausgangsmaterials, welches den Gewebefaktor enthält, in einem Tensid-haltigen Medium und Gewinnen eines Gewebefaktor-haltigen Extrakts,
   - Reinigen des Gewebefaktor-haltigen Extrakts durch Affinitätschromatographie unter Verwendung von immobilisierten Antikörpern, welche gegen den Gewebefaktor gerichtet sind,
   - Abtrennen von vorhandenem Tensid und
   - Relipidisieren des Gewebefaktors durch Zugabe von hochgereinigten Phospholipiden in einem Medium, welches keine Schwermetallionen enthält.

2. Verfahren nach Anspruch 1, dadurch gekennzeichnet, daß Antikörper der gleichen Spezies wie diejenige des Ausgangsmaterials verwendet werden.

3. Verfahren nach Anspruch 2, dadurch gekennzeichnet, daß es sich um monoklonale Antikörper handelt.

4. Verfahren nach einem der Ansprüche 1 bis 3, dadurch gekennzeichnet, daß die hochgereinigten Phospholipide in vesikulärer Form zugegeben werden.

5. Verfahren nach einem der Ansprüche 1 bis 4, dadurch gekennzeichnet, daß als hochgereinigte Phospholipide eine Mischung enthaltend Phosphatidylserin, Phosphatidylcholin, Phosphatidylethanolamin oder Phosphatidylsäure oder Mischungen davon eingesetzt werden.

6. Verfahren nach einem der Ansprüche 1 bis 5, dadurch gekennzeichnet, daß eine Behandlung zur Inaktivierung von Viren durchgeführt wird.

7. Aktive Gewebefaktor-Präparation von biogener Herkunft, die entweder als klare Lösung vorliegt oder in lyophilisierter Form zu einer klaren Lösung rekonstituierbar ist, erhältlich nach einem Verfahren nach einem der Ansprüche 1 bis 6.

8. Gewebefaktor-Präparation nach Anspruch 7, dadurch gekennzeichnet, daß sie einen Gewebefaktor aus Kaninchen oder Rind enthält.

9. Gewebefaktor-Präparation nach einem der Ansprüche 7 oder 8, dadurch gekennzeichnet, daß sie einen Tensidgehalt von weniger als 0,5 Gew.-%, vorzugsweise von weniger als 0,1 Gew.-%, aufweist.

10. Gewebefaktor-Präparation nach einem der Ansprüche 7 bis 9, dadurch gekennzeichnet, daß sie einen Gehalt an Calciumionen im Bereich zwischen 5 mM und 50 mM aufweist.

11. Gewebefaktor-Präparation nach einem der Ansprüche 7 bis 10, dadurch gekennzeichnet, daß sie einen Puffer, wie z.B. einen Tris-NaCl-, Imidazol- oder Hepes-Glycin-Puffer, beinhaltet.

12. Verwendung einer Gewebefaktor-Präparation nach einem der Ansprüche 7 bis 11 zur Herstellung eines Thromboplastinreagens.

13. Verwendung einer Gewebefaktor-Präparation nach einem der Ansprüche 7 bis 11 zur Herstellung eines Reagens zur Bestimmung von Faktoren der extrinsischen Gerinnung.

14. Thromboplastinreagens enthaltend eine Gewebefaktor-Präparation nach einem der Ansprüche 7 bis 11 und Calciumionen.

15. Set zur Herstellung einer Gewebefaktor-Präparation nach einem der Ansprüche 7 bis 11 enthaltend die Komponenten

    a) affinitätschromatographisch gereinigten Gewebefaktor und
    b) hochgereinigte Phospholipide.

16. Set nach Anspruch 15, enthaltend beide Komponenten in lyophilisierter Form und weiters eine wässerige Lösung frei von Schwermetallionen zur Rekonstitution.

## Claims

1. A method of preparing an active tissue factor preparation,
   characterised by the following steps:

   - extracting a biogenic starting material comprising the tissue factor, in a tenside-containing medium, and recovering a tissue factor-containing extract,
   - purifying the tissue factor-containing extract by affinity chromatography using immobilized antibodies directed against the tissue factor,
   - separating tenside present, and
   - relipidizing the tissue factor by adding highly purified phospholipids in a medium which does not contain heavy metal ions.

2. A method according to claim 1, characterised in that antibodies of the same species as that of the starting material are used.

3. A method according to claim 2, characterised in that they are monoclonal antibodies.

4. A method according to any one of claims 1 to 3,
   characterised in that the highly purified phospholipids are added in vesicular form.

5. A method according to any one of claims 1 to 4,
   characterised in that a mixture comprising phosphatidyl serine, phosphatidyl choline, phosphatidyl ethanol amine or phosphatidylic acid or mixtures thereof are used as highly purified phospholipids.

6. A method according to any one of claims 1 to 5,
   characterised in that a treatment for the inactivation of viruses is carried out.

7. An active tissue factor preparation of biogenic origin, present either as a clear solution or which, in lyophilized form is capable of being reconstituted to a clear solution, obtainable by a method according to any one of claims 1 to 6.

8. A tissue factor preparation according to claim 7,
   characterised in that it comprises a tissue factor from rabbit or a bovine tissue factor.

9. A tissue factor preparation according to any one of claims 7 or 8, characterised in that it has a tenside content of less than 0.5 % by weight, preferably of less than 0.1 % by weight.

**10.** A tissue factor preparation according to any one of claims 7 to 9, characterised in that it has a content of calcium ions ranging between 5 mM and 50 mM.

**11.** A tissue factor preparation according to any one of claims 7 to 10, characterised in that it comprises a buffer, such as, e.g., a Tris-NaCl, imidazole or Hepes-glycine buffer.

**12.** The use of a tissue factor preparation according to any one of claims 7 to 11, for preparing a thromboplastin reagent.

**13.** The use of a tissue factor preparation according to any one of claims 7 to 11 for preparing a reagent for determining factors of extrinsic coagulation.

**14.** A thromboplastin reagent comprising a tissue factor preparation according to any one of claims 7 to 11 and calcium ions.

**15.** A kit for preparing a tissue factor preparation according to any one of claims 7 to 11 comprising the components

       a) a tissue factor purified by affinity chromatography, and
       b) highly purified phospholipids.

**16.** A kit according to claim 15 comprising both components in lyophilized form and, furthermore, an aqueous solution free from heavy metal ions for reconstitution.

**Revendications**

**1.** Procédé de production d'une préparation de facteur tissulaire active, caractérisé par les étapes suivantes :

-    extraction d'un produit de départ biogène qui contient le facteur tissulaire, dans un milieu contenant un tensioactif et obtention d'un extrait contenant le facteur tissulaire,
-    purification de l'extrait contenant le facteur tissulaire par chromatographie d'affinité au moyen d'anticorps immobilisés qui sont dirigés contre le facteur tissulaire,
-    séparation du tensioactif présent et
-    relipidisation du facteur tissulaire par addition de phospholipides hautement purifiés dans un milieu qui ne contient pas d'ions de métaux lourds.

**2.** Procédé selon la revendication 1, caractérisé en ce que l'on utilise des anticorps de la même espèce que ceux du produit de départ.

**3.** Procédé selon la revendication 2, caractérisé en ce qu'il s'agit d'anticorps monoclonaux.

**4.** Procédé selon l'une des revendications 1 à 3, caractérisé en ce que les phospholipides hautement purifiés sont ajoutés sous forme vésiculaire.

**5.** Procédé selon l'une des revendications 1 à 4, caractérisé en ce que l'on utilise comme phospholipides hautement purifiés un mélange contenant de la phosphatidylsérine, de la phosphatidylcholine, de la phosphatidyléthanolamine ou de l'acide phosphatidique ou des mélanges de ceux-ci.

**6.** Procédé selon l'une des revendications 1 à 5, caractérisé en ce qu'un traitement pour l'inactivation des virus est réalisé.

**7.** Préparation de facteur tissulaire active d'origine biogène qui est présente sous forme de solution limpide ou qui peut être reconstituée sous forme lyophilisée en une solution limpide, qui peut être obtenue par un procédé selon l'une des revendications 1 à 6.

**8.** Préparation de facteur tissulaire selon la revendication 7, caractérisée en ce qu'elle contient un facteur tissulaire de lapin ou de boeuf.

**9.** Préparation de facteur tissulaire selon l'une des revendications 7 et 8, caractérisée en ce qu'elle présente une teneur en tensioactif inférieure à 0,5 % en masse, de préférence inférieure à 0,1 % en masse.

10. Préparation de facteur tissulaire selon l'une des revendications 7 à 9, caractérisée en ce qu'elle présente une teneur en ions calcium dans le domaine situé entre 5 mM et 50 mM.

11. Préparation de facteur tissulaire selon l'une des revendications 7 à 10, caractérisée en ce qu'elle contient un tampon, comme par exemple un tampon Tris-NaCl, imidazole ou Hepes-glycine.

12. Utilisation d'une préparation de facteur tissulaire selon l'une des revendications 7 à 11 pour la production d'un réactif thromboplastinique.

13. Utilisation d'une préparation de facteur tissulaire selon l'une des revendications 7 à 11 pour la production d'un réactif pour déterminer des facteurs de la coagulation extrinsèque.

14. Réactif thromboplastinique contenant une préparation de facteur tissulaire selon l'une des revendications 7 à 11 et des ions calcium.

15. Ensemble pour la production d'une préparation de facteur tissulaire selon l'une des revendications 7 à 11 contenant les composants

   a) facteur tissulaire purifié par chromatographie d'affinité et
   b) phospholipides hautement purifiés.

16. Ensemble selon la revendication 15 contenant les deux composants sous forme lyophilisée et en outre une solution aqueuse exempte d'ions de métaux lourds pour la reconstitution.

Fig.1

11